# EUROPEAN PATENT SPECIFICATION

(11) **EP 0 956 068 B1**
(45) Date of publication and mention of the grant of the patent: **29.08.2007**
(21) Application number: 97927994.0
(22) Date of filing: 10.06.1997
(51) Int. Cl.: A61M 16/00, A62B 7/00, B23K 1/00, B23K 11/16, F24H 1/10, F24J 3/00, H05B 3/58, A61M 16/08, F24H 3/04

(54) **VENTILATION TUBE, PARTICULARLY FOR MEDICAL DEVICES**
BELÜFTUNGSROHR INSBESONDERE FÜR MEDIZINISCHE VORRICHTUNGEN
TUBE DE VENTILATION, DESTINE PARTICULIEREMENT A DES DISPOSITIFS MEDICAUX

(30) Priority: 13.06.1996 IT MI960435 U
(43) Date of publication of application: 17.11.1999
(73) Proprietor: MALLINCKRODT, INC., St. Louis, MO 63134 (US)
(72) Inventor: GIBERTONI, Lucio, I-41037 Mirandola (IT); BERGAMASCHI, Paolo, I-41033 Concordia (IT)
(74) Representative: Carpmaels & Ransford
(86) International application number: PCT/US1997/009770
(87) International publication number: WO 1997/047348

(56) References cited:
- DE-C- 669 254
- GB-A- 2 173 274
- US-A- 3 891 007
- US-A- 4 686 354
- US-A- 5 357 948
- US-A- 5 454 061

## Description

### Technical Field

The present invention relates to a ventilation tube particularly for medical devices.

### Background art

In many medical devices and particularly in ventilation tubes for intensive care, in ventilation tubes at the outlet of humidifiers and the like, it is conventionally necessary to heat the gaseous substance, generally air, that circulates inside the tube.

An electric resistor is currently used to solve the problem, said resistor being located inside the plastic tube, so as to heat the air to prevent condensation of the moisture contained therein.

This solution entails considerable drawbacks, since any burns caused by the electric resistor on the tube wall might generate fumes which would be fed to the patient, with the obvious associated problems.

Another drawback is also constituted by the fact that a resistor located inside the tube is unable to provide for uniform heating of the inside walls of the tube and therefore cold spots may form which constitute condensation regions for the moisture that is present in ventilation cases, accordingly creating regions in which water accumulates and which, in addition to obstructing the useful cross-section of the tube, also constitute regions for bacterial growth.

In order to solve the above problem, ventilation tubes are already commercially available in which an external wire is provided which is wound in a spiral around the outer wall of the tube; this solution is considerably expensive and also causes problems, since it is necessary to provide for an additional tube in order to sheath the resistor, both owing to electrical insulation problems and in order to prevent excessively hot spots from remaining on the outside, possibly leading to problems for users.

This structure has the drawback that it is very heavy and that it considerably reduces the transparency, lightness and flexibility characteristics which are typical of a PVC tube. Another drawback resides in the costs, since the sheathing of the tube, in addition to increasing the amount of plastics being used, requires much more complex and expensive production technologies than the ordinary spiral tube.

U.S. Patent No. 5,454,061 describes a helically ribbed plastic tubing incorporating an electrically conductive heating wire.

### Disclosure of the Invention

The present invention is defined in the appendant claims. The aim of the present invention is to solve the above problems, by providing a ventilation tube particularly for medical devices which allows to provide uniform heating on the entire surface of the tube affected by the flow of the gaseous substance, thus helping to eliminate the regions where liquid might condense.

Within the scope of this aim, a particular object of the invention is to provide a ventilation tube in which the heating element is not located inside the tube, so that any burns do not produce fumes which are fed directly to the patient, and is furthermore capable of maintaining the flexibility, lightness and transparency characteristics of the spiral tube substantially unchanged.

Another object of the present invention is to provide a ventilation tube which allows to obtain tubes which are already equipped with the heating element, with the possibility of performing extremely simple connections.

Another object of the present invention is to provide a ventilation tube particularly for medical devices, which can be easily obtained starting from commonly commercially available elements and materials and which is also competitive from a merely economical point of view.

### Brief Description of the Drawings

This aim, these objects, and others which will become apparent hereinafter are achieved by a ventilation tube particularly for medical devices, illustrated only by way of non-limitative example in the accompanying drawings, wherein:
figure 1 is a partially cutout schematic view of a portion of ventilation tube according to the present invention;
figure 2 is an enlarged-scale sectional view of the tube;
figure 3 is a view of the detail of the embedding of the electric wires.

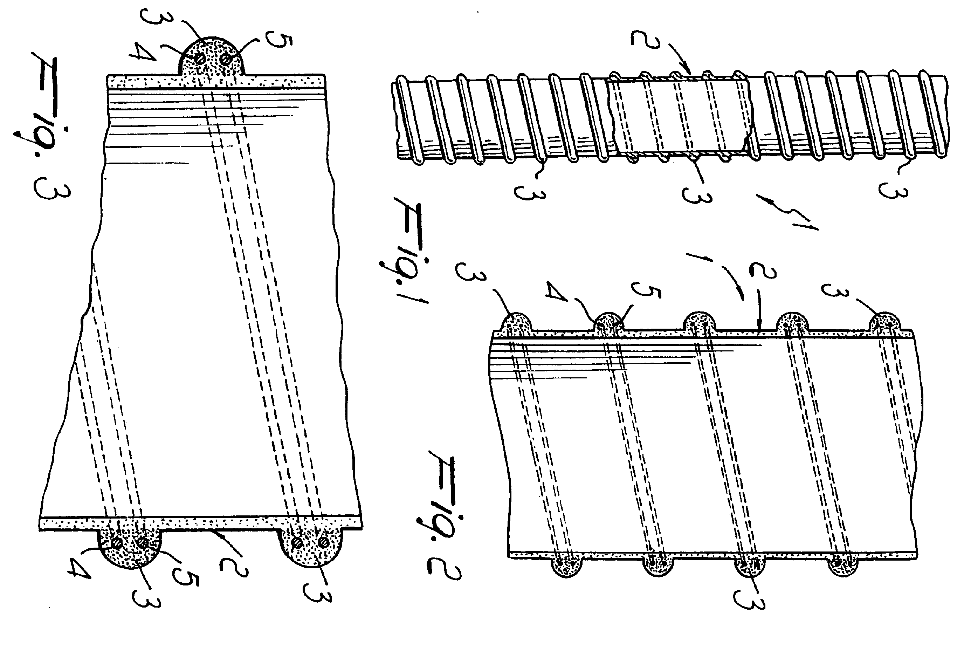

### Ways of carrying out the Invention

With reference to the above figures, the ventilation tube particularly for medical devices, according to the invention, is generally designated by the reference numeral 1 and comprises a tubular body 2 made of flexible plastics, such as PVC, which is externally provided with ridges formed by a spiral ridge 3 which is rigidly coupled to the outer surface of the tubular body 2 since it is formed in a single extrusion process and is made of a more rigid plastics which allows to constitute the conventional spiral of a tube.

A particularity of an embodiment of the invention is constituted by the fact that two mutually spaced electric wires 4 and 5 are embedded directly inside the spiral ridge 3 and constitute an electric resistor.

The two electric wires are not in mutual contact and in practice the material that forms the ridge constitutes the electrical insulation for the copper wires.

Advantageously, two electric wires are used so that by simply joining them at one end an electric resistor is obtained with the possibility of connecting the remaining two ends to the electric power source.

The tubular body is advantageously made of transparent plastics, so that the tube remains transparent, and the direct insertion of the copper wires in the spiral ridge allows to considerably contain the weight of the tube as well as its manufacturing cost.

Another advantage obtained is constituted by the fact that the uniform distribution of the wires that form the electric resistor along the surface allows uniform heating in all points of the electrical surface while having excellent electrical safety characteristics, since the electric wires are directly embedded in the ridge, which is monolithic with the tube.

In practice, the materials employed, so long as they are compatible with the specific use, as well as the contingent shapes and dimensions, may be any according to requirements.

## Claims

1. A ventilation tube (1), particularly for medical devices, comprising:
an elongate and flexible plastic conduit (2);
a helical plastic ridge (3) formed on an outside diameter of said conduit;
at least one wire (4, 5) in said ridge (3) to form an electric resistor for heating a gas that flows, in use, inside the tube (1) ; and
wherein said flexible plastic conduit (2) is formed with said ridge (3)
**characterised by** said wire (4, 5) being embedded directly in said ridge which is formed with said flexible plastic conduit as a single extrusion.

2. A ventilation tube (1) according to claim 1, **characterized in that** said at least one ridge (3) is formed monolithically with said tubular body (2) and are made of a material which is more rigid than the material that constitutes said conduit.

3. A ventilation tube (1) according to the preceding claims, **characterized in that** said at least one ridge (3) is constituted by an element which is arranged in a spiral pattern on the outer surface of the tube (1).

4. A ventilation tube (1) particularly for medical devices, **characterized in that** it comprises one or more of the described and/or illustrated characteristics.

5. A process for making a ventilation tube according to anyone of claims 1 to 4, comprising:
simultaneously extruding an elongate flexible extruded plastic conduit and a helical plastic ridge formed on an outside diameter of said conduit; and
inserting wire conductors uniformly in said ridge while said ridge is being extruded to form an electric resistor for heating a gas that flows inside the tube.

## Patentansprüche

1. Belüftungsrohr (1) insbesondere für medizinische Geräte, umfassend:
einen länglichen und flexiblen Kunststoffschlauch (2);
einen helixförmigen Kunststoffgrat (3), der auf einem Außendurchmesser des Schlauchs ausgebildet ist;
mindestens einen Draht (4, 5) in dem Grat (3) zum Bilden eines elektrischen Widerstands zum Beheizen eines Gases, das im Betrieb im Innern des Rohrs (1) strömt; und
wobei der flexible Kunststoffschlauch (2) mit dem Grat (3) geformt wird, **dadurch gekennzeichnet, dass** der Draht (4, 5) direkt in den Grat eingebettet ist, der mit dem flexiblen Kunststoffschlauch als eine gemeinsame Extrusion geformt wird.

2. Belüftungsrohr (1) nach Anspruch 1, **dadurch gekennzeichnet, dass** der mindestens eine Grat (3) mit dem röhrenförmigen Körper (2) einstückig ausgebildet ist und aus einem Werkstoff hergestellt ist, der steifer als der Werkstoff ist, aus dem der Schlauch besteht.

3. Belüftungsrohr (1) nach einem der vorhergehenden Ansprüche, **dadurch gekennzeichnet, dass** der mindestens eine Grat (3) aus einem Element besteht, das in einem Spiralenmuster auf der Außenoberfläche des Rohrs (1) angeordnet ist.

4. Belüftungsrohr (1) insbesondere für medizinische Geräte, **dadurch gekennzeichnet, dass** es eines oder mehrere der beschriebenen und/oder veranschaulichten Merkmale aufweist.

5. Verfahren zum Herstellen eines Belüftungsrohrs gemäß einem der Ansprüche 1 bis 4, umfassend:
gleichzeitiges Extrudieren eines länglichen flexiblen extrudierten Kunststoffschlauchs und eines helixförmigen Kunststoffgrats, der auf einem Außendurchmesser des Schlauchs ausgebildet ist; und
gleichmäßiges Einbringen von Drahtleitern in den Grat, während der Grat extrudiert wird, zum Bilden eines elektrischen Widerstands zum Beheizen eines in dem Rohr strömenden Gases.

## Revendications

1. Tube de ventilation (1), en particulier pour les dispositifs médicaux, comprenant :
un conduit en plastique allongé et souple (2) ;
une strie en plastique hélicoïdale (3) formée sur un diamètre extérieur dudit conduit ;
au moins un fil (4, 5) dans ladite strie (3) pour former une résistance électrique pour chauffer un gaz qui circule, lors de l'utilisation, à l'intérieur du tube (1) ; et dans lequel ledit conduit en plastique souple (2) est formé avec ladite strie (3) ;
**caractérisé par le fait que** ledit fil (4, 5) est intégré directement dans ladite strie qui est formée avec ledit conduit en plastique souple en tant qu'extrusion unique.

2. Tube de ventilation (1) selon la revendication 1, **caractérisé en ce que** ladite au moins une strie (3) est formée monolithiquement avec ledit corps tubulaire (2) et est constituée d'un matériau qui est plus rigide que le matériau qui constitue ledit conduit.

3. Tube de ventilation (1) selon les revendications précédentes, **caractérisé en ce que** ladite au moins une strie (3) est constituée par un élément qui est agencé en une configuration hélicoïdale sur la surface extérieure du tube (1).

4. Tube de ventilation (1) en particulier pour les dispositifs médicaux, **caractérisé en ce qu'**il comprend une ou plusieurs des caractéristiques décrites et/ou illustrées.

5. Procédé pour fabriquer un tube de ventilation selon l'une quelconque des revendications 1 à 4, comprenant :
l'extrusion simultanée d'un conduit en plastique extrudé allongé et souple et d'une strie en plastique hélicoïdale formée sur un diamètre extérieur dudit conduit ; et
l'insertion de fils conducteurs uniformément dans ladite strie tandis que ladite strie est extrudée pour former une résistance électrique pour chauffer un gaz qui circule à l'intérieur du tube.
